# EUROPÄISCHE PATENTSCHRIFT

(11) **EP 2 282 783 B1**
(45) Veröffentlichungstag und Bekanntmachung des Hinweises auf die Patenterteilung: **12.03.2014**
(21) Anmeldenummer: 09745659.4
(22) Anmeldetag: 30.04.2009
(51) Int. Cl.: A61L 2/10

(54) **VORRICHTUNG ZUM STERILISIEREN VON BEHÄLTNISVERSCHLÜSSEN**
DEVICE FOR STERILIZING CONTAINER CLOSURES
DISPOSITIF POUR STÉRILISER DES FERMETURES DE RÉCIPIENTS

(30) Priorität: 15.05.2008 DE 102008023797
(43) Veröffentlichungstag der Anmeldung: 16.02.2011
(73) Patentinhaber: KRONES Aktiengesellschaft, 93073 Neutraubling (DE)
(72) Erfinder: SCHMATZ, Stefan, 93170 Bernhardswald (DE)
(74) Vertreter: Hannke, Christian
(86) Internationale Anmeldenummer: PCT/EP2009/055263
(87) Internationale Veröffentlichungsnummer: WO 2009/138326

(56) Entgegenhaltungen:
- EP-A1- 0 277 505
- EP-A1- 1 518 565
- WO-A1-99/12434
- DE-A1- 19 520 925
- GB-A- 2 364 299
- US-A- 5 928 607
- US-A1- 2006 011 263

## Beschreibung

Die vorliegende Erfindung bezieht sich auf eine Vorrichtung zum Sterilisieren von Behältnisverschlüssen und ist insbesondere zum Sterilisieren von Verschlüssen für Kunststoff- oder Glasflaschen geeignet.

Aus dem Stand der Technik ist bekannt, Behältnisse, insbesondere auch unter sterilen Bedingungen, mit einem Getränk abzufüllen und anschließend die Behältnisse mit Behältnisverschlüssen zu verschließen. Dabei ist auch darauf zu achten, dass diese Behältnisverschlüsse das Produkt selbst nicht kontaminieren können. Aus diesem Grunde ist es bekannt, die Verschlüsse, bevor sie auf die Behältnisse aufgebracht werden, zu sterilisieren. Eine Möglichkeit der Sterilisierung besteht darin, dass die Verschlüsse mit Gas wie beispielsweise H₂O₂ - Dampf oder mit Flüssigkeiten wie Desinfektionsmitteln beaufschlagt werden. Für diese Art der Sterilisierung sind jedoch aufwändige Transport-, Abdichtungs- sowie Behandlungseinrichtungen erforderlich. Zusätzlich werden in derartigen Verfahren Chemikalien eingesetzt, teuer sind, meist nicht recycelt werden können, die Gesundheit des Anlagenbedieners gefährden und mit erheblichem Aufwand wieder von den Verschlüssen entfernt werden müssen. Eine weitere Möglichkeit der Sterilisation besteht darin, die Behältnisverschlüsse mit sterilisierender Strahlung, insbesondere ultravioletter Strahlung (UV - Strahlung), zu beaufschlagen.

Aus der US 2,095,502 ist eine Sterilisationsvorrichtung bekannt. Dabei werden zu reinigende Objekte einer sterilisierenden Lichtstrahlung ausgesetzt. Die WO 01/17891 A1 beschreibt eine Vorrichtung zum Abfüllen von Behältern, wobei die Verschlüsse vor Eintritt in einen Reinraum gereinigt werden und wobei die Reinigung auch mittels einer Strahleinrichtung, welche UV-Strahlung abgibt, erfolgen kann.

Aus der DE 44 07 183 A1 sind ein Verfahren und eine Vorrichtung zum Sterilisieren von Behältern bekannt. Dabei werden Behälter getaktet in Druckanordnung unter eine entsprechend angeordnete Gruppe von permanent eingeschalteten abgeschirmten UV-Strahlern gefahren. Anschließend werden die UV-Strahler in die Flaschen verbracht, wodurch eine Innensterilisation dieser Behältnisse erfolgen kann und wiederum anschließend gereinigt.

Die WO 02/36437 A1 beschreibt Verfahren und Vorrichtungen bei denen Verpackungen einer UV-Strahlung ausgesetzt werden. Die WO 92/18170 offenbart ein Sterilisierverfahren wobei das zu sterilisierende Material einer UV-Strahlung ausgesetzt wird.

Die WO 03/021173 A1 beschreibt eine Bestrahlungsvorrichtung zum Bestrahlen von Objekten mit UV-Strahlung, wobei eine längliche UV-Lampe zum Ausstrahlen von UV-Strahlung und ein länglicher Reflektor verwendet werden, um die Strahlung auf die Behältnisse zu richten. Derartige Vorrichtungen kommen im industriellen Einsatz im Zielgebiet der erfindungsgemäßen Vorrichtung bevorzugt zum Einsatz.

Die DE 202 01 493 U1 beschreibt eine Bestrahlungsvorrichtung zum Bestrahlen eines Objekts mit Strahlung im ultravioletten oder sichtbaren Bereicht. Dabei werden eine oder mehrere Leuchtdioden verwendet, welche das zu bestrahlende Objekt beleuchten.

Aus der EP 1 614 630 A1 sind ein Verfahren und eine Vorrichtung zum Sterilisieren von Behältern mit UV-Strahlung bekannt. Dabei soll insbesondere die Behälterinnenfläche mit UV-Strahlung behandelt werden, wobei UV-Strahlung einer außerhalb des Behälters befindlichen UV-Quelle in den Behälterinnenraum eingeführt wird.

Aus der US 2006/001263 ist eine Anlage zum Befüllen von Behältnissen bekannt. Dabei werden die Behältnisse mittels ultravioletter Strahlung sterilisiert, bevor sie mit Flüssigkeit befüllt werden. Diese Strahlung wird dabei ausgehend von einer Strahlungsquelle über optische Wellenleiterelemente in die Behälter eingeführt.

Die WO 99/12434 beschreibt eine Vorrichtung zum deaktivieren von Mikroorganismen. Diese Vorrichtung ist dabei auf die Sterilisation von bandartigen Materialen gerichtet.

Die aus dem Stand der Technik bekannten Vorrichtungen weisen dabei das Problem auf, dass die Lichterzeugungsquelle oft nur schwer zugänglich ist, da sie innerhalb der Anlage verbaut werden. Da die UV-Quellen, auch bei einem durch Betriebsstörungen bedingten Anlagenstillstand, nicht abgeschaltet werden können, um ein zeitintensives Wieder-Inbetriebnehmen zu vermeiden, werden die UV-Quellen bekannterweise mit Abschirmungseinrichtungen - sogenannten "Shuttern" - ausgestattet, welche den Strahlungsaustritt auf die zu bestrahlenden Objekte während des Stillstandes verhindern. Ohne diese Abschirmungseinrichtungen würden die zu bestrahlenden Objekte einer zu intensiven Bestrahlung ausgesetzt, da sie Anlagenstillstandsbedingt sich nicht an der Strahlungsquelle vorbeibewegen, sondern stehen. Durch das genannte Abschirmen entsteht im Gegenzug ein Wärmestau innerhalb der Strahlungsquelle, was zur Folge der Notwendigkeit einer Kühlungseinrichtung führt. Diese Kühlungseinrichtungen sind aufwändig und insbesondere in Rein- oder Sterilräumen nicht erwünscht, da deren hygienischer Zustand schlecht kontrollierbar ist und z.B. bei Kühlgebläsen unerwünschte Luftverwirbelungen im Sterilraum entstehen. Diese Kühlungseinrichtung wird zwar durch die Vorrichtung aus oben genannter WO 01/17891 A1 vermieden, jedoch werden durch die Bestrahlung vor Eintritt in den Sterilraum aufwändige Schleusen und eingehauste Transporteinrichtungen notwendig.

Aufgrund der komplexen Lichtstreuungsverhältnisse im Inneren ist es weiterhin nötig, Transportrinnen für die Verschlüsse gut zu verbauen, um Streustrahlungen zu vermeiden. Dadurch wird ein Wechsel dieser Rinnen, der insbesondere bei Anlagen notwendig ist, welche vielerlei verschiedenartige Behältnisverschlüsse auf Behältnisse aufbringen müssen, zeitaufwändig, was für den Anlagenbetreiber zu vermehrtem Ausfall an Produktionszeit führt und die Produktivität der Anlage reduziert. Weiterhin ist es auch nicht möglich, bekannte Vorrichtungen zum Reinigen von Behältnissen auf Vorrichtungen zum Reinigen von Behältnisverschlüssen zu übertragen, da Behältnisverschlüsse zwar einerseits keine großflächige Desinfektion erfordern wie beispielsweise Behältniswände , andererseits jedoch im Bereich des Gewindes eine sehr komplexe und dadurch schwer zu reinigende Struktur aufweisen. Aus den dargestellten Gründen ist bislang die Reinigung von Behältnisverschlüssen mittels UV - Strahlung insbesondere in Sterilräumen und insbesondere in Sterilanwendungen eher unüblich, sondern es wird überwiegend auf die Verwendung von Desinfektionsmitteln zurückgegriffen.

Der vorliegenden Erfindung liegt daher die Aufgabe zugrunde, eine Vorrichtung und ein Verfahren zur Verfügung zu stellen, welches eine Vereinfachung der aus dem Stand der Technik bekannten Vorrichtungen erlaubt. Insbesondere soll eine verbesserte Zugänglichkeit der Transportvorrichtungen für die Behältnisverschlüsse erreicht werden. Auch sollen Wechsel dieser Transportvorrichtungen leichter durchführbar sein. Diese Aufgaben werden erfindungsgemäß durch die Gegenstände der unabhängigen Ansprüche erreicht, vorteilhafte Ausführungen und Weiterbildungen sind Gegenstand der Unteransprüche.

Eine erfindungsgemäße Verrichtung zum Sterilisieren von Behältnisverschlüssen weist eine Transporteinrichtung auf, welche die Behältnisverschlüsse entlang eines vorgegebenen Transportpfads transportiert. Weiterhin weist die Vorrichtung eine Einhausung auf, welches die Transporteinrichtung wenigstens abstandsweise umgibt, sowie eine Lichtquelle, die die Behältnisverschlüsse während des Transportes entlang des Transportpfades mit ultravioletter Strahlung beaufschlagt. Erfindungsgemäß ist die Lichtquelle außerhalb der Einhausung angeordnet und die ultraviolette Strahlung wird in das Innere der Einhausung und auf die Behältnisverschlüsse geleitet, bzw. in das Innere der Einhausung geführt. Die Einhausung umgibt die erfindungsgemäße Vorrichtung wenigstens teilweise, d.h. erfindungsgemäß kann die Vorrichtung in einem Rein- oder Sterilraum ebenso stehen, wie die Einhausung durch einen dem Fachmann bekannten Maschinenschutz dargestellt werden kann.

Damit wird vorgeschlagen, die Lichtquelle selbst außerhalb einer Einhausung beziehungsweise außerhalb eines Sterilraumes anzuordnen. Auf diese Weise ist die Lichtquelle wie insbesondere eine UV-Lampe, leicht zur Reparatur und Montagezwecken zugänglich. Weiterhin kann gleichzeitig eine Lüftung für diese Lichtquelle anders als im Stand der Technik ebenfalls außerhalb der Einhausung vorgesehen werden, so dass es im Inneren der Einhausung nicht zu ungewollten Luftbewegungen kommt.

Weiterhin ist es auch nicht nötig, die Transporteinrichtung bzw. Verschlussrinne einzukapseln und zu verbauen, sodass ein Wechsel der Rinne leichter durchführbar ist. Die erfindungsgemäße Vorrichtung ist damit auch leicht bei bestehenden Anlagen nachrüstbar, und auch von den Größen der Verschlussrinnen unabhängig. Weiterhin wird das im Stand der Technik bekannte Problem minimiert, dass es beim Anlagenstillstand zu einer Erwärmung der Verschlüsse durch die Abwärme der Lichtquelle kommen kann. Im Falle eines Anlagenstillstandes oder einer Störung kann auch die Verwendung von Prozessluft zur Kühlung der Lichtquellen entfallen.

Während im Stand der Technik für unterschiedliche Lichtquellen unterschiedliche Sonderkonstruktionen erforderlich sind, ist es nunmehr möglich die (in ihrer Positionierung unkritische) Lichtquelle mit standardmäßigen Bauteilen anzuordnen.

Vorzugsweise weist die Vorrichtung einen Lichtleiter auf, der die Strahlung von der Lichtquelle zu den Behältnisverschlüssen leitet. Auf diese Weise ist es möglich, das Licht auch über gekrümmte bzw. verbaute Wege von der Lichtquelle zu den Behältnisverschlüsse zu befördern. Durch die Verwendung von Lichtleitern kann auch der Abschirmungsaufwand reduziert werden. Es wäre jedoch möglich, dass die Lichtquelle das Licht direkt auf die sterilisierenden Behältnisverschlüsse richtet.

Weiterhin ist eine Einkoppelvorrichtung vorgesehen, um die ultraviolette Strahlung in den Lichtleiter einzukoppeln. Bei dieser Einkoppelvorrichtung handelt es sich vorzugsweise um eine Linse oder ein Prisma.

Bei einer weiteren vorteilhaften Ausführungsform ist die Transporteinrichtung eine Transportschiene oder Transportrinne, entlang derer die Behältnisverschlüsse vorzugsweise unter Wirkung der Schwerkraft gleiten. Dabei bildet besonders bevorzugt die Transportschiene einen Staubereich aus, innerhalb dessen die Behältnisverschlüsse unmittelbar nacheinander liegend befördert werden. Im Gegensatz zur Transportvorrichtungen für Behältnisse, welche üblicherweise getaktet und mit vorbestimmten Abständen zueinander transportiert werden, wird hier vorgeschlagen, die Behältnisverschlüsse im Bereich einer Staustrecke anzustauen, um auf diese Weise eine vergleichsweise langsame Bewegung der Behältnisverschlüssen zu erreichen, damit diese effizient auch an kritischen Stellen beispielsweise dem Bereich ihres Innengewindes sterilisiert werden können. Im Gegensatz zu Vorrichtungen, welche Behältnisse sterilisieren, werden die Verschlüsse bevorzugt auch nicht einzeln gegriffen.

Bei einer weiteren vorteilhaften Ausführungsform weist die Vorrichtung eine Austrittseinrichtung auf, welche die Lichtstrahlung unmittelbar auf die Behältnisverschlüsse richtet. Dabei werden insbesondere diejenigen Bereiche der Behältnisverschlüsse, welche die Gewinde aufweisen, sterilisiert. Vorzugsweise ist die Transporteinrichtung lediglich im Bereich dieser Austrittseinrichtung eingehaust. Es wäre jedoch auch möglich, die Austrittseinrichtung, bei der es sich beispielsweise auch um eine Linse oder ein Prisma handelt, so nah an die Behältnisse heranzuführen, dass auf die Einhausung vollständig verzichtet werden kann.

Dabei führt die Transportschiene die Behältnisverschlüsse vorzugsweise derart, dass deren Innenbereich (also auch das Gewinde) der Austrittseinrichtung zugewandt ist.

Bei einer weiteren vorteilhaften Ausführungsform weist die Austrittseinrichtung eine Streueinrichtung auf, welche das auf die Behältnisverschlüsse gelangende Licht streut. Auf diese Weise können größere Flächen der Behältnisverschlüsse sterilisiert werden. Bei dieser Streueinrichtung handelt es sich besonders bevorzugt ebenfalls um ein Prisma. Gleichwohl kann jedoch die Strahlung insbesondere auf die kritischen Zonen der Behältnisverschlüsse gelenkt werden.

Bei einer weiteren vorteilhaften Ausführungsform weist die Vorrichtung mehrere Austrittseinrichtungen auf, mit denen von derselben Lichtquelle stammende Strahlen auf die Behältnisse gerichtet werden, wobei diese Austrittseinrichtungen an unterschiedlichen Stellen des Transportpfades der Behältnisse angeordnet sind. Auf diese Weise ist es in besonders bequemer Weise möglich, unterschiedliche Bereiche der Behältnisverschlüsse zu sterilisieren. Dabei kann, obwohl die Sterilisation an unterschiedlichen Bereichen des Transportpfades stattfindet, die gleiche Lichtquelle verwendet werden. So ist es beispielsweise möglich, die Behältnisverschlüsse mit einer ersten Austrittseinrichtung an ihrem Außenumfang zu sterilisieren und mit einer weiteren Austrittseinrichtung die Innenbereiche der Behältnisverschlüsse zu sterilisieren. Dabei sind vorzugsweise zwischen allen Austrittseinrichtungen und der Lichtquelle jeweils Lichtleiter, und insbesondere aber nicht ausschließlich Glasfaserkabeln, vorgesehen.

Vorzugsweise werden die Behältnisverschlüsse innerhalb eines Sterilraumes transportiert und dieser Sterilraum ist von der Einhausung umgeben. Insbesondere für Sterilanwendungen eignet sich die vorliegende Erfindung besonders, da bei derartigen Anwendungen auch die-Größe der Flächen innerhalb des Sterilraumes gering gehalten werden soll. Die Einhausung dichtet dabei bevorzugt den Sterilraum vollständig gegenüber der Umgebung ab. Auch können in dem Sterilraum andere Druckverhältnisse herrschen als in der Umgebung.

Bei einer weiteren vorteilhaften Ausführungsform ist die Transporteinrichtung innerhalb der Einhausung wenigstens abschnittsweise nicht abgeschirmt. Dies bedeutet, dass in diesem Bereich die Transporteinrichtung beziehungsweise die Rinne sehr gut zugänglich beziehungsweise sehr gut auswechselbar ist.

Die vorliegende Erfindung ist weiterhin auf eine Anlage zum Verschließen von Behältnissen mit Behältnisverschlüssen gerichtet, die eine Vorrichtung der oben beschriebenen Art und eine stromabwärts bezüglich dieser Vorrichtung angeordnete Verschließeinrichtung zum Anbringen der Behältnisverschlüsse an den Behältnissen aufweist. Diese Verschließeinrichtung weist bevorzugt ein oder mehrere Verschließelemente auf, welche die Behältnisverschlüsse auf die Behältnisse aufdrehen oder anderweitig anbringen.

Bevorzugt ist die Verschließeinrichtung zum Verschließen der Behältnisse innerhalb des Sterilraums, in dem auch die Transporteinrichtung für die Behältnisverschlüsse vorgesehen ist, angeordnet. Weiterhin weist die Anlage bevorzugt eine weitere Sterilisationseinheit zum Sterilisieren der Behältnisse auf. Auch diese weitere Einheit kann innerhalb des Sterilraums (in der auch die Transporteinrichtung vorgesehen ist) untergebracht sein. Dabei ist es möglich, die Behältnisse selbst ebenfalls mit UV - Strahlung zu sterilisieren. Auch wäre es möglich, zum Sterilisieren der Behältnisse die gleiche Lichtquelle zu verwenden wie zum Sterilisieren der Behältnisverschlüsse.

Die vorliegende Erfindung ist weiterhin auf ein Verfahren zum Sterilisieren von Behältnisverschlüssen gerichtet, wobei die Behältnisverschlüsse innerhalb einer Einhausung entlang eines vorgegebenen Transportpfades transportiert werden und während dieses Transportes durch eine Lichtquelle mit ultravioletter Strahlung zu deren Sterilisation beaufschlagt werden. Erfindungsgemäß wird die Strahlung außerhalb des Gehäuses erzeugt und in das Gehäuse geleitet. Bevorzugt werden die Behältnisverschlüsse nach deren Sterilisation auf Behältnissen angebracht bzw. aufgeschraubt.

Vorzugsweise werden die Behältnisverschlüsse an mehreren Bereichen entlang des Transportpfades mit ultravioletter Strahlung beaufschlagt.

Weitere Vorteile und Ausführungsformen ergeben sich aus den beigefügten Zeichnungen:
Darin zeigen:
   - Fig.1: eine schematische Darstellung zur Veranschaulichung der Erfindung; und
   - Fig. 2: eine detaillierte Darstellung einer erfindungsgemäßen Vorrichtung.

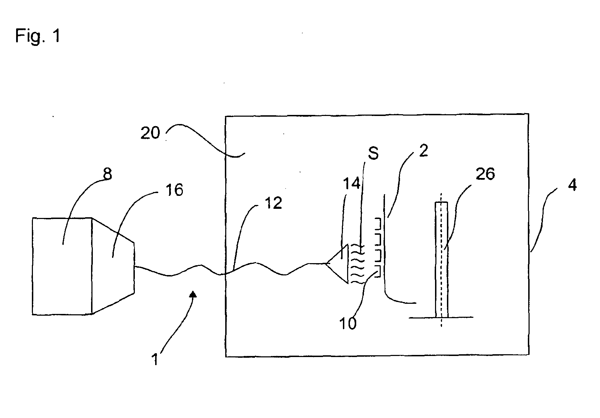

Fig. 1 zeigt eine schematische Darstellung einer erfindungsgemäßen Vorrichtung 1. Diese Vorrichtung weist eine Einhausung 4 auf, innerhalb derer ein Reinraum beziehungsweise ein Sterilraum 20 gebildet wird. Vorzugsweise ist dieser Sterilraum 20 im Wesentlichen luftbeziehungsweise gasdicht gegenüber der Umgebung 25 abgeschlossen. Im Inneren der Einhausung 4 ist eine Transporteinrichtung 2 vorgesehen, mittels derer die Behältnisverschlüsse 10 transportiert werden. Dabei werden, wie in Fig.1 gezeigt, die Behältnisverschlüsse derart transportiert, dass deren Öffnungen direkt von ultravioletter Strahlung S bestahlt werden können.

Das Bezugszeichen 8 bezieht sich auf eine Lichtquelle, die über ein Prisma und einen Lichtleiter 12 die ultraviolette Strahlung in den Sterilraum 20 einstrahlt. Dabei sind (nicht gezeigte) Öffnungen in der Einhausung 4 vorgesehen, durch welche hindurch der Lichtleiter in das Innere der Einhausung 4 geführt wird. Vorzugsweise sind auch Dichtungseinrichtungen vorgesehen, welche den Übergang zwischen dem Lichtleiter 12 und der entsprechenden Einhausungswand abdichten.

Das Bezugszeichen 14 bezieht sich auf eine Austrittsvorrichtung, welche die von dem Lichtleiter 12 kommende Strahlung flächig auf die Behältnisverschlüsse 10 richtet, wobei, wie oben erwähnt, die Innenbereiche der Behältnisverschlüsse 10 dieser Austrittseinrichtung 14 zugewandt sind.

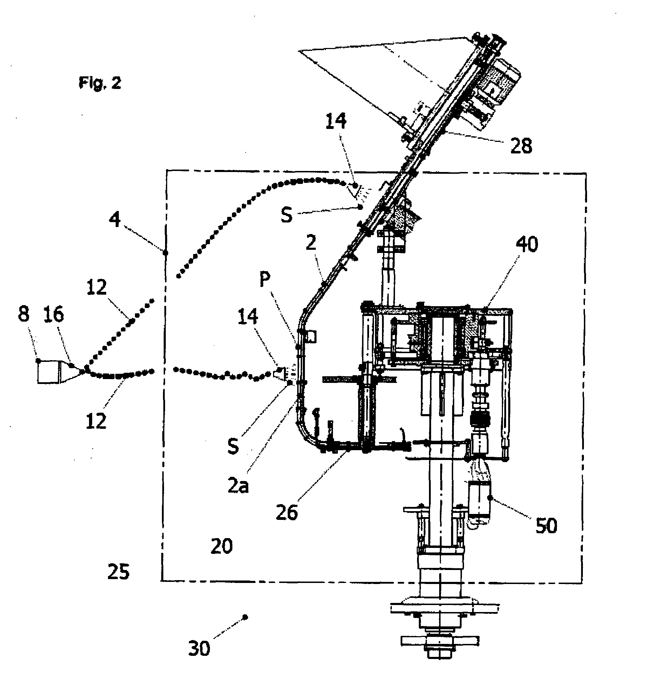

Fig. 2 zeigt eine detailliertere Darstellung einer erfindungsgemäßen Vorrichtung 1 bzw. einer Anlage 30 zum Verschließen von Behältnissen 50 mit Behältnisverschlüssen. Man erkennt hier, dass die Transporteinrichtung 2 als Rinne ausgeführt ist, innerhalb derer die Verschlüsse (nicht gezeigt) gleiten können. Dabei ist ein vertikaler Abschnitt 2a dieser Rinne vorgesehen, innerhalb derer die Verschlüsse vertikal nach unten gleiten. Bevorzugt ist in diesem Bereich auch eine Staustrecke vorgesehen, sodass sich hier die Behältnisverschlüsse 10 aufstauen. Erfindungsgemäß kann statt einer vertikalen Förderung durch Schwerkraft auch jegliches bekannte Förderungsverfahren zur Förderung der Verschlüsse eingesetzt werden wie z. B. ein pneumatischer Transport durch Sterilluft oder Einrichtungen zum mechanischen Vorschub der Verschlüsse.

Weiterhin sind, wie in Fig. 2 gezeigt, zwei Austrittseinrichtungen 14 vorgesehen, welche in unterschiedlichen Bereichen der Transporteinrichtung 2 angeordnet sind. Das Bezugszeichen 28 bezieht sich auf ein Sortierwerk, innerhalb dessen die Behältnisverschlüsse sortiert und beispielsweise auch in einer Ebene angeordnet werden. In diesen Bereich ist ebenfall eine Bestrahlung mit UV-Strahlung besonders gut geeignet, um eine Sterilisierung der Behältnisverschlüsse zu erreichen.

Das Bezugszeichen P kennzeichnet den Transportpfad der Behältnisverschlüsse. Man erkennt, dass beide Austrittseinrichtungen 14 von der gleichen Lichtquelle, die außerhalb des Gehäuses 4 angeordnet ist versorgt werden. Das Bezugszeichen 16 bezieht sich auf ein Prisma, welches zur Lichtbündelung dient.

An die Transportschiene schließt sich eine Bildaufnahmeeinrichtung beziehungsweise Inspektionseinrichtung 22 an, welche Bilder der Behältnisverschlüsse aufnimmt. Das Bezugszeichen 40 bezieht sich im Gesamten auf eine Verschließeinrichtung, welche die Behältnisverschlüsse auf zu verschließenden Behältnissen 50 anbringt. Es wird darauf hingewiesen, dass auch die Verschließeinrichtung 40 innerhalb des Gehäuses 4 angeordnet ist.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind.

## Patentansprüche

1. Vorrichtung (1) zum Sterilisieren von Behältnisverschlüssen (10) mit einer Transporteinrichtung (2), welche die Behältnisverschlüsse (10) entlang eines vorgegebenen Transportpfades (P) transportiert, mit einer Einhausung (4), welche die Transporteinrichtung (2) wenigstens abschnittsweise umgibt, und mit einer Lichtquelle (8), welche die Behältnisverschlüsse (10) während des Transports entlang des Transportpfads (P) mit ultravioletter Strahlung (S) beaufschlagt,
**dadurch gekennzeichnet, dass**
die Lichtquelle (8) außerhalb der Einhausung (4) angeordnet ist und die ultraviolette Strahlung (S) in das Innere der Einhausung (4) geleitet wird.

2. Vorrichtung (1) nach Anspruch 1,
**dadurch gekennzeichnet, dass**
die Vorrichtung einen Lichtleiter (12) aufweist, der die Strahlung von der Lichtquelle (8) zu den Behältnisverschlüssen (10) leitet.

3. Vorrichtung (1) nach wenigstens einem der vorangegangenen Ansprüche,
**dadurch gekennzeichnet, dass**
die Transporteinrichtung (2) eine Transportschiene ist, entlang derer die Behältnisverschlüsse (10) unter Wirkung der Schwerkraft entlang gleiten.

4. Vorrichtung (1) nach Anspruch 3,
**dadurch gekennzeichnet, dass** die Transportschiene einen Staubereich ausbildet, innerhalb dessen die Behältnisverschlüsse (10) unmittelbar aneinanderliegend befördert werden.

5. Vorrichtung (1) nach Anspruch 1 oder 2,
**dadurch gekennzeichnet, dass**
die Transporteinrichtung (2) ein Lufttransport ist.

6. Vorrichtung (1) nach Anspruch 1 oder 2,
**dadurch gekennzeichnet, dass**
die Transporteinrichtung (2) eine mechanische Vorschubvorrichtung aufweist.

7. Vorrichtung (1) nach wenigstens einem der vorangegangenen Ansprüche,
**dadurch gekennzeichnet, dass**
die Vorrichtung (1) eine Austrittseinrichtung (14) aufweist, welche die Lichtstrahlung unmittelbar auf die Behältnisverschlüsse (10) richtet.

8. Vorrichtung (1) nach Anspruch 7,
**dadurch gekennzeichnet, dass**
an der Austrittseinrichtung (14) eine Streueinrichtung angeordnet ist, welche das auf die Behältnisverschlüsse (10) gelangende Licht streut.

9. Vorrichtung (1) nach wenigstens einem der vorangegangenen Ansprüche 7 - 8
**dadurch gekennzeichnet, dass**
die Vorrichtung (1) mehrere Austrittseinrichtungen (14) aufweist, mit denen von derselben Lichtquelle (8) stammende Strahlung auf die Behältnisse (10) gerichtet wird, wobei die Austrittseinrichtungen (14) an unterschiedlichen Stellen des Transportpfads der (P) Behältnisse (10) angeordnet sind.

10. Vorrichtung (1) nach wenigstens einem der vorangegangenen Ansprüche,
**dadurch gekennzeichnet, dass**
die Behältnisverschlüsse (10) innerhalb eines Sterilraums (20) transportiert werden und dieser Sterilraum (20) von der Einhausung (4) umgeben ist.

11. Vorrichtung (1) nach wenigstens einem der vorangegangenen Ansprüche,
**dadurch gekennzeichnet, dass**
die Transporteinrichtung (2) innerhalb der Einhausung (4) wenigstens abschnittweise nicht abgeschirmt ist.

12. Anlage (30) zum Verschließen von Behältnissen (50) mit Behältnisverschlüssen (10) mit einer Vorrichtung (1) nach wenigstens einem der vorangegangenen Ansprüche und einer stromabwärts bezüglich dieser Vorrichtung (1) angeordneten Verschließeinrichtung (40) zum Anbringen der Behältnisverschlüsse (10) an den Behältnissen (50).

13. Anlage (30) nach Anspruch 12,
**dadurch gekennzeichnet, dass**
die Verschließeinrichtung (40) innerhalb des Sterilraums (20) angeordnet ist.

14. Verfahren zum Sterilisieren von Behältnisverschlüssen (10), wobei die Behältnisverschlüsse (10) innerhalb einer Einhausung (4) entlang eines vorgegebenen Transportpfads (P) transportiert und während dieses Transports von einer Lichtquelle (8) mit ultravioletter Strahlung zu deren Sterilisation beaufschlagt werden,
**dadurch gekennzeichnet, dass**
die Strahlung außerhalb des Gehäuses (4) erzeugt und in die Einhausung (4) geleitet wird.

15. Verfahren nach Anspruch 14,
**dadurch gekennzeichnet, dass**
die Behältnisverschlüsse (10) nach deren Sterilisation auf Behältnissen (50) angebracht werden.

## Claims

1. An apparatus (1) for sterilizing container closures (10) with a conveying device (2) which conveys the container closures (10) along a pre-determined conveying path (P), with a housing (4) which surrounds the conveying device (2) at least in sections, and with a light source (8) which acts upon the container closures (10) with ultraviolet radiation (S) during the conveying along the conveying path (P), **characterized in that** the light source (8) is arranged outside the housing (4) and the ultraviolet radiation (S) is directed into the interior of the housing (4).

2. An apparatus (1) according to claim 1, **characterized in that** the apparatus has a light conductor (12) which directs the radiation from the light source (8) to the container closures (10).

3. An apparatus (1) according to at least one of the preceding claims, **characterized in that** the conveying device (2) is a conveying rail, along which the container closures (10) slide under the action of gravity.

4. An apparatus (1) according to claim 3, **characterized in that** the conveying rail forms a back-up area, inside which the container closures (10) are conveyed lying one immediately against each other.

5. An apparatus (1) according to claims 1 or 2, **characterized in that** the conveying device (2) is an air conveyor.

6. An apparatus (1) according to claims 1 or 2, **characterized in that** the conveying device (2) has a mechanical advancing apparatus.

7. An apparatus (1) according to at least one of the preceding claims, **characterized in that** the apparatus (1) has an outlet device (14) which aims the light radiation directly onto the container closures (10).

8. An apparatus (1) according to claim 7, **characterized in that** a scattering device, which scatters the light arriving at the container closures (10), is arranged on the outlet device (14).

9. An apparatus (1) according to at least one of the preceding claims 7 to 8, **characterized in that** the apparatus (1) has a plurality of outlet devices (14) by which radiation originating from the same light source (8) is aimed at the containers (10), wherein the outlet devices (14) are arranged at different points on the conveying path of the (P) containers (10).

10. An apparatus (1) according to at least one of the preceding claims, **characterized in that** the container closures (10) are conveyed inside a sterile room (20) and this sterile room (20) is surrounded by the housing (4).

11. An apparatus (1) according to at least one of the preceding claims, **characterized in that** the conveying device (2) is not screened off inside the housing (4) at least sectionwise.

12. A plant (30) for closing containers (50) with container closures (10) with an apparatus (1) according to at least one of the preceding claims and a closure device (40) arranged downstream with respect to this apparatus (1) for attaching the container closures (10) to the containers (50).

13. A plant (30) according to claim 12, **characterized in that** the closure device (40) is arranged inside the sterile room (20).

14. A method of sterilizing container closures (10), wherein the container closures (10) are conveyed inside a housing (4) along a pre-determined conveying path (P) and during this conveying are acted upon by a light source (8) with ultraviolet radiation to sterilize them, **characterized in that** the radiation is produced outside the housing (4) and is introduced into the housing (4).

15. A method according to claim 14, **characterized in that** the container closures (10) are attached to containers (50) after they have been sterilized.

## Revendications

1. Système (1) pour la stérilisation de bouchons pour des récipients (10), avec un dispositif de transport (2) qui convoie les bouchons pour des récipients (10) le long d'un chemin de transport (P) défini, avec une enceinte (4) qui entoure au moins partiellement le dispositif de transport (2), et avec une source lumineuse (8) qui applique un rayonnement ultraviolet (S) sur les bouchons pour des récipients (10) pendant le convoyage le long du chemin de transport (P), **caractérisé en ce que**
la source lumineuse (8) est disposée à l'extérieur de l'enceinte (4) et le rayonnement ultraviolet (S) est dirigé vers l'intérieur de l'enceinte (4).

2. Système (1) selon la revendication 1, **caractérisé en ce que**
ledit système comporte un guide d'ondes lumineuses (12) qui conduit le rayonnement depuis la source lumineuse (8) vers les bouchons pour des récipients (10).

3. Système (1) selon au moins une des revendications précédentes, **caractérisé en ce que**
le dispositif de transport (2) est un rail de transport sur lequel glissent les bouchons pour des récipients (10) sous l'effet de la gravité.

4. Système (1) selon la revendication 3, **caractérisé en ce que**
le rail de transport forme une zone d'accumulation à l'intérieur de laquelle les bouchons pour des récipients (10) sont convoyés en étant directement accolés l'un contre l'autre.

5. Système (1) selon la revendication 1 ou 2, **caractérisé en ce que**
le dispositif de transport (2) est un convoyage pneumatique.

6. Système (1) selon la revendication 1 ou 2, **caractérisé en ce que**
le dispositif de transport (2) comporte un dispositif d'avance mécanique.

7. Système (1) selon au moins une des revendications précédentes, **caractérisé en ce que**
ledit système (1) comporte un dispositif de sortie (14) qui oriente directement le rayonnement lumineux vers les bouchons pour des récipients (10).

8. Système (1) selon la revendication 7, **caractérisé en ce qu'**
un dispositif de diffusion est monté sur le dispositif de sortie (14), lequel diffuse la lumière arrivant sur les bouchons pour des récipients (10).

9. Système (1) selon au moins une des revendications 7 et 8, **caractérisé en ce que**
ledit système (1) comporte plusieurs dispositifs de sortie (14), par lesquels un rayonnement provenant de la même source lumineuse (8) est orienté vers les bouchons pour récipients (10), lesdits dispositifs de sortie (14) étant situés à des emplacements différents sur le chemin de transport (P) des récipients (10).

10. Système (1) selon au moins une des revendications précédentes, **caractérisé en ce que**
les bouchons pour des récipients (10) sont convoyés à l'intérieur d'un espace stérile (20) et **en ce que** ledit espace stérile (20) est délimité par l'enceinte (4).

11. Système (1) selon au moins une des revendications précédentes, **caractérisé en ce que**
le dispositif de transport (2) n'est au moins partiellement pas isolé à l'intérieur de l'enceinte (4).

12. Installation (30) pour l'obturation de récipients (50) avec des bouchons pour des récipients (10), comportant un système (1) selon au moins une des revendications précédentes et un dispositif d'obturation (40) pour appliquer les bouchons pour récipients (10) sur les récipients (50), situé en aval dudit système (1).

13. Installation (30) selon la revendication 12, **caractérisée en ce que**
le dispositif d'obturation (40) est situé à l'intérieur de l'espace stérile (20).

14. Procédé de stérilisation de bouchons pour récipients (10), lesdits bouchons pour des récipients (10) étant convoyés à l'intérieur d'une enceinte (4) le long d'un chemin de transport (P) défini, et étant soumis pendant ce convoyage à un rayonnement ultraviolet d'une source lumineuse (8) pour leur stérilisation, **caractérisé en ce que**
le rayonnement est généré à l'extérieur de l'enceinte (4) et dirigé vers l'intérieur de l'enceinte (4).

15. Procédé selon la revendication 14, **caractérisé en ce que**
les bouchons pour des récipients (10) sont appliqués sur des récipients (50) après leur stérilisation.
